# EUROPEAN PATENT APPLICATION

(11) **EP 2 887 063 A1**
(43) Date of publication of application: **24.06.2015**
(21) Application number: 13807625.2
(22) Date of filing: 06.06.2013
(51) Int. Cl.: G01N 33/48

(54) **DISPOSABLE DIAGNOSTIC CARD AND ELECTRONIC READING DEVICE**

(30) Priority: 19.06.2012 ES 201230957
(71) Applicant: Laboratorios Alpha San Ignacio Pharma, S.L., 50008 Zaragoza (ES)
(72) Inventor: PREHN GALLO, Ricardo, E-08192 Sant Quirze del Valles (Barcelona) (ES); MUÑOZ PASCUAL, Francisco Javier, E-08192 Sant Quirze del Valles (Barcelona) (ES); CALAVIA CALVO, Daniel, E-5003 Zaragoza (ES); RONCALES POZA, Miguel, E-28050 Madrid (ES); JUEZ JIMENEZ, Alejandro, E-22005 Huesca (ES); MALO YAGÜE, Laura, E-50017 Zaragoza (ES); FERNANDEZ LEDESMA, Luis José, E-50430 Maria de Huerva (Zaragoza) (ES)
(74) Representative: Covadonga Fernández-Vega Feijoo, Maria
(86) International application number: PCT/ES2013/070370
(87) International publication number: WO 2013/190155

(57) **Abstract**

The invention relates to a disposable diagnostic card for detecting an analyte in a fluid sample, comprising a base with a support surface for supporting a reagent strip and a sample receiving chamber; and a cover placed tightly on the base with an opening over the chamber of the base for introducing the sample, and a window over part of the reagent strip for observing a reaction thereon. An end of the reagent strip is bent and inserted into the sample receiving chamber, the sample in the chamber being movable by capillary action through the reagent strip from said bent end. The invention also relates to an electronic reading device having a housing for inserting a diagnostic card, optical reading means for reading a change in color on the reagent strip, and display means for visually presenting the results of the reading.

## Description

### FIELD OF THE INVENTION

The present invention relates to the field of medical diagnostic devices, and more specifically to a disposable diagnostic card for detecting an analyte in a body fluid sample by means of reagent strips.

### PRIOR ART

A wide variety of diagnostic kits is known in the art the purpose of which is to detect a medically useful analyte in a patient's body fluid sample, such as a saliva or blood sample, for example. The kits known today generally comprise an object used for collecting the sample, for example, in the case of saliva samples, a stick with an absorbent material at the tip intended for being introduced in the patient's mouth. They also comprise a second object which stores the sample and allows dispensing it on an analysis cartridge, and finally the analysis cartridge itself. Furthermore, in the case of obtaining too little fluid samples, some kits also comprise an object storing a type of buffer in which the absorbent material with the sample is introduced to dissolve it for the purpose of obtaining a sufficient sample volume.

Most of these detection kits resort to using reagent strips for performing said detection. These reagent strips are based on the so-called lateral-flow principle. The main problem with reagent strip-based diagnostic kits known today arises from the difficulty in obtaining homogeneous sample dispersion along said reagent strips.

When the sample volume obtained is too large, the base of the cartridge comprising the reagent strips overflows and as a result the sample is unevenly distributed, incorrectly wetting the reagent strips, invalidating the test result.

On the other hand, if the sample volume is too little, it is insufficient for obtaining acceptable results in the diagnostic reaction. There is therefore a need to provide an additional element containing a buffer to dilute the sample, and the additional dilution step itself, which increases testing time and the cost of the actual diagnostic kit.

In fact, the problem due to obtaining an insufficient sample volume is very common in certain situations. For example, in the case of narcotics detection in saliva samples, a situation known as "dry mouth syndrome" often occurs in which the subject is not capable of producing sufficient saliva to perform detection. This syndrome is enhanced by the use of alcohol, drugs and the like, which greatly complicates controls on narcotics use.

Diabetics also use reagent strip-based diagnostic kits of this type for determining blood glucose level. Under certain circumstances, a diabetic subject must perform diagnostic test several times a day by means of a pricking a fingertip to obtain a blood sample that must then be applied on the reagent strip. Over time, it becomes difficult to obtain a sufficient volume of blood sample from the fingertip for detection purposes.

Therefore, there is still a need in the art for a system which allows detecting an analyte in a body fluid sample (such as saliva or blood) by means of reagent strips, provides homogeneous sample distribution along said reagent strips and is not affected by too large or too little fluid sample volumes.

### DISCLOSURE OF THE INVENTION

In a first aspect, the present invention discloses a disposable diagnostic card for detecting an analyte in a body fluid sample. The diagnostic card comprises a base and a cover. The base has a support surface for supporting at least one reagent strip and a fluid sample receiving chamber. The cover is in turn placed tightly on the base and has an opening over the chamber of the base for inserting the fluid sample, and a window over at least part of the at least one reagent strip for observing a reaction thereon.

An end of the at least one reagent strip supported by the base of the diagnostic card is bent, such that it is inserted into the sample receiving chamber. The fluid sample inserted in the chamber can therefore move by capillary action along the at least one reagent strip from said bent end, this movement of the fluid sample through the reagent strip always being homogeneous and sufficient regardless of the sample volume introduced in the receiving chamber.

In a second aspect, the present invention also relates to an electronic reading device having at least one housing for inserting a diagnostic card according to the first aspect of the present invention. The reading device also has optical reading means for reading a change in color in the at least one reagent strip of the diagnostic card, and display means for visually presenting a result of the reading of said at least one reagent strip.

### BRIEF DESCRIPTION OF THE DRAWINGS

The present invention will be better understood in reference to the following drawings which illustrate a preferred embodiment of the invention which is provided by way of example and must not be interpreted as limiting the invention in any way.
Figure 1 shows a perspective view of a disposable diagnostic card according to the preferred embodiment of the present invention.
Figure 2 shows a perspective view of the base of the diagnostic card of Figure 1.
Figure 3 shows a perspective view of the cover of the diagnostic card of Figure 1.
Figure 4 shows a perspective view of the cap of the diagnostic card of Figure 1.

### DETAILED DISCLOSURE OF PREFERRED EMBODIMENTS

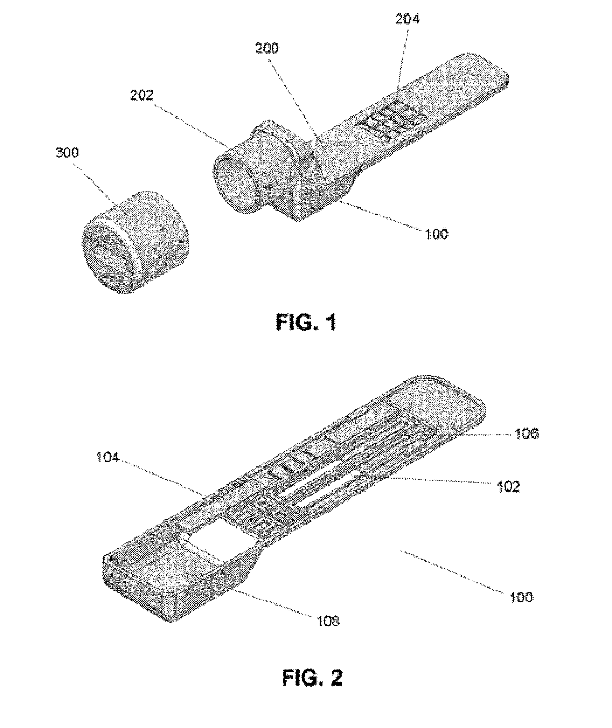

A disposable diagnostic card according to the preferred embodiment of the present invention for detecting narcotics in a saliva sample from a subject will now be described in reference to the attached drawings. As can be seen in Figure 1, the diagnostic card is formed by a base (100), a cover (200) and a cap (300). The saliva sample is introduced in the card by means of a suitable swab through a hole (202) of the cover (200) partially covered by the cap (300). The saliva sample that has been introduced will then react with reagent strips supported by the base (100) and said reaction will cause a change in color in the reagent strips that can be seen through a window (204) present in the cover (200).

In reference to Figure 2, it can be seen that the base (100) of the disposable diagnostic card has a support surface (102) supporting in this case three reagent strips (104) (only one is shown in Figure 1 for the sake of simplicity). It can be seen that this support surface (102) furthermore has partition walls (106) partitioning individual tracks for each reagent strip (104), such that the strips (104) are held on the support surface (102) better.

The base (100) also comprises a chamber (108) intended for receiving the saliva sample from the subject. Although it is not shown in the attached drawings for the sake of simplicity, an end of the reagent strips (104) is bent such that it is inserted in the sample receiving chamber (108). Said bent end is preferably dyed for pigment diffusion with the saliva sample along the reagent strip (104), thus marking bands such that it allows interpreting the result.

Therefore, when the saliva sample is introduced in the chamber (108), said saliva sample can move by capillary action along the reagent strip (104), always in a homogeneous manner, regardless of the volume of said saliva sample. In fact, in the case of obtaining a too large saliva sample volume, the sample is distributed homogeneously since it only occurs from the bent end of the reagent strip (204). On the other hand, in the case of obtaining a too little saliva sample volume, sufficient dispersion by capillary action also occurs from the end of the reagent strip (104) since said end is in contact with the bottom of the chamber (108), which is used for accumulating the saliva sample on a smaller surface.

According to the preferred embodiment of the present invention, the bent end of the reagent strip (104) is separated from the bottom of the chamber (108), i.e., it does not contact it. The height of said bent end of the reagent strip (104) with respect to the bottom of the chamber (108) determines a saliva sample volume that will be collected in said chamber (108) and can be subjected, if necessary, to a contrast test in a certified laboratory to check the result of a detection reaction.

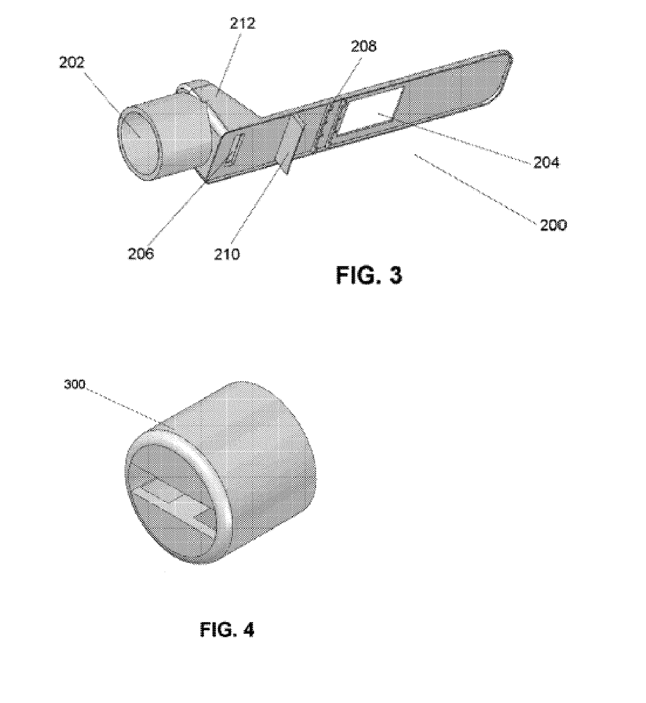

In reference to Figure 3, a cover (200) intended for being placed tightly on the base (100) described above can be seen. The cover (200) has an opening (206), in this case in the form of a groove, arranged such that it is located over the chamber (108) of the base (100) for introducing the saliva sample. According to a preferred embodiment of the invention, the bent end of the reagent strip (104) is located below said opening (206), such that said reagent strip (104) absorbs part of the saliva sample as it falls through said opening (206).

As mentioned previously, the cover (200) also has a window (204) arranged such that it is located over at least part of the reagent strips (104) to thus enable observing a reaction thereon. Said window (204) is preferably covered with a suitable transparent material, such as transparent plastic, such that it protects the reagent strips (104) located under it from external contaminants, while at the same time it allows viewing a reaction occurring in said reagent strips (104).

It must be observed that the cover (200) according to this preferred embodiment of the invention also has holding elements (208) projecting from its lower surface for additionally fixing the reagent strips (104) in place. Finally, a vertical wall (210) extends from a lower surface of the cover (200) towards the inside of the sample receiving chamber (108). The person skilled in the art will therefore understand that when said vertical wall (210) is introduced on a chamber (108) in a base (100) already comprising the reagent strips (104), it will cause the reagent strips (104) to bend in a desired manner towards the bottom of the sample receiving chamber (108). This facilitates assembling the diagnostic card since it is not necessary to bend the reagent strips (104) before placing them on the support surface (102) of the base (100).

On the opening (206) for introducing the saliva sample, the cover (200) has a hollow extension (212) with a hole (202), in this case a cylindrical hole, arranged for inserting a sample collecting swab (not shown in the drawings) therein. Though not shown in the attached drawings, the person skilled in the art will understand that said hollow extension (212) also has an inner wall against which said swab is pressed, said wall extending vertically over the opening (206) for introducing the sample. Therefore, when the swab is pressed against said wall the saliva sample absorbed by the swab is extracted and allowed to flow through the wall to the chamber (108) where it will accumulate.

Finally in reference to Figure 4, a cap (300) intended for covering the hole (202) of the cover (200) is seen. On the other hand, though not shown in the attached drawings, according to the preferred embodiment of the present invention the cap (300) comprises a stick with a sample collecting swab made of an absorbent and biocompatible material coupled therein. The disposable diagnostic card according to the preferred embodiment of the present invention therefore comprises all the elements necessary for performing the desired detection test, such that it does not require adding any external element, whereby reducing the number and volume of elements that must be transported as well as the testing time.

The present invention also relates to an electronic reading device intended for being used together with a disposable diagnostic card such as that described above in this document. The electronic reading device of the present invention therefore has at least one housing for inserting a diagnostic card as described above. According to a preferred embodiment of the invention, the electronic reading device only has one housing for inserting one diagnostic card at a time, such that its volume and weight is reduced, therefore obtaining a portable device. However, according to another embodiment of the invention the electronic reading device has a plurality of said housings, such that several diagnostic cards can be read at the same time. In the last case, it is a desktop device suitable for use in an analysis center or laboratory, for example.

Furthermore, the electronic reading device of the present invention also comprises optical reading means suitable for reading a change in color in the reagent strip or strips of the diagnostic card inserted in its corresponding housing, and display means (for example a screen) for visually presenting a result of the reading of said reagent strip.

Although the present invention has been described in reference to a preferred embodiment thereof, people skilled in the art will understand that various modifications and variations can be applied to the described embodiment without departing from the essence and scope of the present invention. For example, although a disposable diagnostic card for detecting narcotic substances in saliva samples has specifically been described, it must be understood that the present invention can be applied to other types of detections, such as, for example, the detection of diseases, such as HIV infection, abnormal cholesterol levels, blood glucose levels, etc. Obviously, the person skilled in the art will understand that in the case of performing detection based on blood samples instead of saliva samples, the diagnostic card of the present invention will not have the swab described above but instead will have puncturing means suitable for withdrawing said blood sample.

Likewise, according to another embodiment of the present invention the cover of the diagnostic card does not have the vertical wall described above, in which case the corresponding end of the reagent strips must be bent manually or by other means before closing the base with the cover.

Other possible embodiments of the present invention include several variations of the description provided above. For example, in certain embodiments of the invention the disposable diagnostic card does not have the cap described above. In such situations, the body fluid sample to be analyzed is inserted directly into the chamber through the opening present in the cover thereof. In these cases, the opening of the cover is preferably closed by means of a closing element which, for example, can either be removed and disposed of at the time of use (such as a removable metallic film, for example) or opened for introducing the fluid sample and then closed again after introducing the sample (for example a lid which can be opened and closed by means of hinges, sliding, etc.).

## Claims

1. A disposable diagnostic card for detecting an analyte in a body fluid sample, **characterized in that** it comprises:
- a base (100) having a support surface (102) for supporting at least one reagent strip (104) and a fluid sample receiving chamber (108); and
- a cover (200) placed tightly on the base (100), having an opening (206) over the chamber (108) of the base (100) for introducing the fluid sample, and a window (204) over at least part of the at least one reagent strip (104) for observing a reaction thereon;
wherein one end of the at least one reagent strip (104) is bent such that it is inserted into the sample receiving chamber (108), the fluid sample in the chamber (108) being movable by capillary action along the at least one reagent strip (104) from said bent end.

2. The diagnostic card according to claim 1, **characterized in that** the bent end of the at least one reagent strip (104) is separated from the bottom of the chamber (108).

3. The diagnostic card according to any of the preceding claims, **characterized in that** the bent end of the at least one reagent strip (104) is located below the opening (206), such that the at least one reagent strip (104) absorbs part of the fluid sample as it falls through said opening (206).

4. The diagnostic card according to any of the preceding claims, **characterized in that** the cover (200) furthermore has a vertical wall (210) extending from a lower surface thereof towards the inside of the sample receiving chamber (108), such that it causes the at least one reagent strip (104) to bend towards the inside of the sample receiving chamber (108).

5. The diagnostic card according to any of the preceding claims, **characterized in that** the support surface (102) of the base (100) furthermore has partition walls (106) for partitioning a plurality of individual tracks for fixing an equivalent plurality of reagent strips (104).

6. The diagnostic card according to any of the preceding claims, **characterized in that** the cover (200) furthermore has holding elements (208) projecting from its lower surface for fixing the at least one reagent strip (104) in place.

7. The diagnostic card according to any of the preceding claims, **characterized in that** the opening (206) of the cover (200) is closed with a closing element which can be removed at the time of use.

8. The diagnostic card according to any of claims 1 to 6, **characterized in that** the opening (206) of the cover (200) is closed with a closing element which can be opened for introducing a fluid sample and closed again after introducing the sample.

9. The diagnostic card according to any of claims 1 to 6, **characterized in that** the cover (200) has on said opening (206) a hollow extension (212) with a hole (202) in which there is inserted a sample collecting swab and a wall against which said swab is pressed, said wall extending vertically over the opening (206) for introducing the sample.

10. The diagnostic card according to claim 9, **characterized in that** it further comprises a cap (300) for closing the hole (202) for inserting the swab of the cover.

11. The diagnostic card according to claim 10, **characterized in that** the cap (300) comprises a stick with a sample collecting swab coupled therein.

12. The diagnostic card according to any of the preceding claims, **characterized in that** the window (204) of the cover is covered with a transparent material.
